# EUROPEAN PATENT APPLICATION

(11) **EP 3 693 353 A1**
(43) Date of publication of application: **12.08.2020**
(21) Application number: 19156412.9
(22) Date of filing: 11.02.2019
(51) Int. Cl.: C07C 17/389, C07C 21/20

(54) **A PROCESS FOR THE PURIFICATION OF FLUORINATED OLEFINS**

(71) Applicant: SOLVAY SA, 1120 Brussels (BE)
(72) Inventor: PITTROFF, Michael, 30539 Hannover (DE); REVELANT, Denis, 69740 GENAS (FR); ZOUGMORE, Talsida, 69340 Francheville (FR)
(74) Representative: Mross, Stefan P.M.

(57) **Abstract**

The present invention relates to a process for the purification of fluorinated olefins using adsorbents with a large average pore size.

## Description

The present invention relates to a process for the purification of fluorinated olefins, such as, especially, hexafluoro-1,3-butadiene, using adsorbents with a large average pore size.

Hexafluoro-1,3-butadiene is a colorless, gaseous unsaturated fluorocarbon with an alternating double bond. It is an etchant showing very high performance for plasma, ion beam, or sputter etching in semiconductor devices manufacturing. Due to its short atmospheric lifetime (< 1day), its negligible global warming potential, and its inertness to the stratospheric ozone layer, hexafluoro-1,3-butadiene is an environmentally compatible gas. Hexafluoro-1,3-butadiene is marketed by Solvay under the brand name Sifren® 46.

Hexafluoro-1,3-butadiene employed in the semiconductor industry must be of extremely high purity. To this end, EP1329442A1 describes a process for the purification of hexafluoro-1,3-butadiene using certain adsorbents with low average pore diameter, particularly molecular sieve 5 Å, since the hexafluoro-1,3-butadiene is apparently excluded from the adsorbent while the impurities are adsorbed and thus, avoiding deleterious decomposition reactions from occurring.

However, there is still a need for improved processes for the purification of hexafluoro-1,3-butadiene. Consequently, one objective of the present invention is to propose an improved process for the purification of hexafluoro-1,3-butadiene. The process of the present invention advantageously lead to a more economical cleaning process, an improved purity, a reduced time of the overall purification process and/or uses cheaper and more readily available adsorbents.

These and other objectives are achieved by the process according to the present invention. The present inventors have surprisingly found that these objectives can be achieved by using at least two different adsorbents of relatively high average pore size.

Accordingly, a first aspect of the present invention concerns a process for the purification of hexafluoro-1,3-butadiene comprising a step wherein a gaseous mixture comprising hexafluoro-1,3-butadiene is contacted with at least two different adsorbents to purify said gas mixture, wherein the at least two different adsorbents each have an average pore size of above 6 Å, as measured by nitrogen adsorption porosimetry.

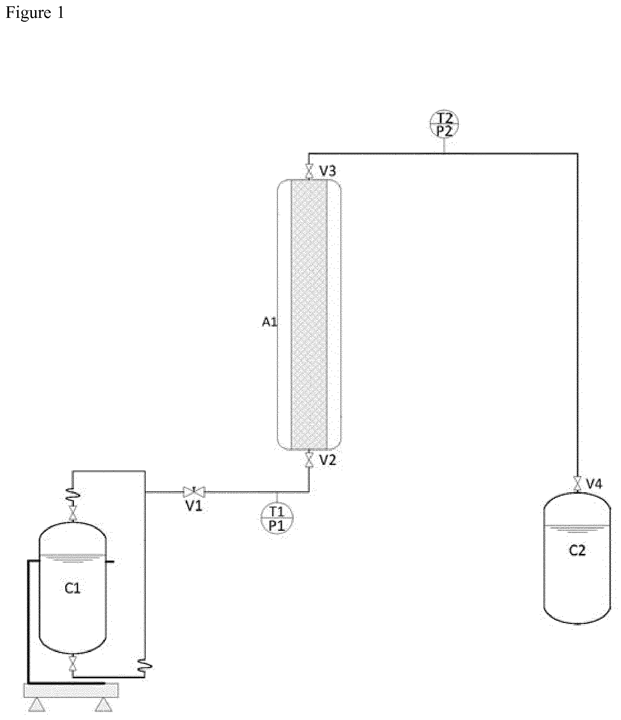

Figure 1 shows a flow diagram of an apparatus for performing the process according to the present invention.

Suitable adsorbents include molecular sieves, preferably alumino silica like zeolites, silica gel, alumina, activated carbon, ceramic membranes and metal-organic frameworks.

The gaseous mixture to be purified may contain various impurities in admixture with the hexafluoro-1,3-butadiene. Such impurities may include water, alcohols, especially isopropanol, hydrohalogenocarbons, especially hydrofluorocarbons. Said impurities may come from the formation of byproducts, from residual solvents, unreacted starting materials and/or partially unreacted starting materials.

The final purity of the hexafluoro-1,3-butadiene achieved by the process according to the invention is equal to greater than 99.9% by volume, preferably equal to greater than 99.95% by volume, more preferably equal to greater than 99.98% by volume, and most preferably equal to greater than 99.99% by volume.

The at least two different adsorbents each have an average pore size of above 6 Å, preferably above 8 Å, more preferably above 10 Å. Also preferably, the at least two different adsorbents each have an average pore size of equal to or below 100 Å, more preferably equal to or below 80 Å, most preferably equal to or below 50 Å. Preferably, at least one of the at least two adsorbents has an average pore size of equal to or above 10 Å, more preferably equal to or above 15 Å, more preferably equal to or above 20 Å. The average pore size can be measured by conventional methods known by the skilled person, such as nitrogen adsorption porosimetry.

Preferably, at least one adsorbent used is silica gel, more preferably silica gel with an average pore size of 10 to 100 Å, more preferably 15 to 50 Å, moste preferably between 10 and 20 Å. A very suitable silica gel includes the Tixosil® range from Solvay as well as SYLOBEAD® SG B125 supplied by Grace.

Also preferably, at least one of the at least two different adsorbents is a zeolite, preferably the zeolite is a gmelinit, mordenite, SAPO-40, ZSM-10 or molecular sieve 13X, specifically molecular sieve 13X.

In a more preferred embodiment, the at least two different adsorbents include at least one zeolite and at least one silica gel, most preferably two adsorbents, one zeolite and one silica gel, are used in the process. Advantageously, the gaseous mixture is first purified with the silica gel and subsequently purified with the zeolite. This implementation of the process is especially preferred because a) the zeolite and the silica gel remove different impurity species and b) the silica gel used in the first purification stage is able to remove certain impurities which were found to be decomposed in the second stage on the zeolite.

Preferably, the process is conducted at an initial pressure of equal to or above 100 mbar (abs.) and equal to or below 2000 mbar (abs.).

Also preferably, the process is conducted at an initial temperature of equal to or above 5°C and equal to or below 40°C.

The term "initial" as used herein is intended to denote that temperature and pressure of the gaseous mixture before coming into contact with the first of the at least two adsorbents.

Also preferably, the flow rate of the gaseous mixture through the adsorbents is set to equal to or above 2 g/min and equal to or below 200 g/min.

In a preferred embodiment, the at least two different adsorbents are present in different zones in the same adsorber cartridge. Thus, only one adsorber cartridge is used in the purification process and the at least two adsorbents are located within the one cartridge in different zones, preferably in subsequent zones allowing the gaseous mixture to be in contact with one adsorbent after the other.

In another preferred embodiment, the adsorbents that are used in the present invention are present in different adsorber cartridges, so that the gaseous mixture can be brought into contact with the adsorbents one after the other and the adsorbents can be regenerated individually.

Advantageously, at least one of the adsorbents used can be pre-treated before being contacted with the gaseous mixture. A usual pre-treatment step can comprise keeping the adsorbent at an elevated temperature, preferably between 150 and 400°C, more preferably from 250 to 350°C, and optionally under reduced pressure. Without being bound by any theory, it is believed that this pre-treatment leads to a decline in the amount of impurities formed by decomposition when the gaseous mixture is being brought into contact with the pre-treated adsorbent. The skilled person knows the suitable conditions for the pre-treatment of a given adsorbent, i.e. the choice of temperature, pressure and time the adsorbent has to be treated so as to achieve removal of moisture and/or adsorbed gases.

The hexafluoro-1,3-butadiene purified according to the present invention can be used neat. However, it is often desired to use the hexafluoro-1,3-butadiene of the present invention as an admixture with other fluorinated etching gases to control the carbon/fluoro ratio of the gas mixture. Additionally, mixtures with suitable inert gases like nitrogen, argon or xenon or with oxygen might be desired.

Accordingly, a further aspect of the present invention is a process for the production of a gas mixture according to the present invention, comprising the process for the purification of hexafluoro-1,3-butadiene described above and subsequently, mixing the purified hexafluoro-1,3-butadiene with a further gas selected from the group consisting of an inert gas, oxygen and another fluorinated etching gas as well as the gas mixture formed in such a process.

The inventive gas mixtures can easily be prepared by condensing or pressing the desired amounts of hexafluoro-1,3-butadiene and any other desired gas into a pressure bottle.

Furthermore, the invention concerns a process for the production of a semiconductor material, a solar panel, a flat panel or a microelectromechanical system, or a process for cleaning the chamber of an apparatus used for semiconductor manufacturing using the hexafluoro-1,3-butadiene purified according to this invention or the gas mixture according to this invention. The preferred use is in the production of a microelectromechanical system.

Should the disclosure of any patents, patent applications, and publications which are incorporated herein by reference conflict with the description of the present application to the extent that it may render a term unclear, the present description shall take precedence.

Figure 1 shows a suitable apparatus for the process of the present invention. Initial tank **C1** contains the crude hexafluoro-1,3-butadiene. The amount of hexafluoro-1,3-butadiene in tank **C1** can be measured by means of a balance. Final tank **C2** is submersed in a cooling bath at -78 °C (mix of dry ice and acetone). Stainless steel tube **A1** contains the adsorbent beds. It has an internal diameter of 18 mm and a length of 406 mm. It is double jacketed and connected to a cooling bath for being able to cool down the bed in case of an exothermic reaction inside. Pressure and temperature of the gaseous mixture before and after tube **A1** are measured. All piping is made of stainless steel.

The following describes a typical sequence of the inventive process using the apparatus as shown in figure 1.

All adsorbents are pre-treated in an oven under argon flow at 250 to 350°C overnight followed by cooling to room temperature. Afterwards they are directly loaded into tube **A1** or stored under dry conditions for later usage.

Once tube **A1** is charged with the required adsorbents, it is installed in the apparatus and the tightness of the apparatus is checked under vacuum.

Afterwards, final tank **C2** is submersed into the cooling bath and tank **C1** is charged with 100 g to 500 g of crude hexafluoro-1,3-butadiene. The pressure in tank **C1** is usually in the range from 1.70 bar to 2.04 bar (abs.).

The crude hexafluoro-1,3-butadiene is then allowed to pass through tube **A1** and the thus purified hexafluoro-1,3-butadiene is collected by condensation in final tank **C2.** The flow is manually controlled from 5 g/min to 7 g/min by adjusting needle valves **V1**, **V2** and **V3** accordingly.

After all crude hexafluoro-1,3-butadiene has been passed through tube **A1**, tank **C2** is isolated by closing valve **V4** and then allowed to warm to room temperature.

A sample of the purified hexafluoro-1,3-butadiene in tank **C2** is analysed and the analysis results are compared to those of the crude hexafluoro-1,3-butadiene.

The following example shall explain the invention in further details, but is not intended to limit the scope of the invention.

### Example 1: Purification of hexafluoro-1,3-butadiene using a combination of silica gel and molecular sieve 13X zeolite

For this trial, tube A1 was first charged with 30.3 g silica gel (SYLOBEAD® SG B125 supplied by Grace, average pore size of 18 Å), which was pre-treated under a stream of argon overnight at 350 °C, at the end of tube **A1** which comes first into contact with the gaseous mixture. Afterwards, the rest of tube A1 was charged with 38.2 g of the zeolite molecular sieve 13X (AxSorb™ 913 supplied by Axens, average pore size of 10 Å), which was pre-treated at 260 °C overnight, at the end of the tube A1 facing final tank **C2**. Thus, tube **A1** was charged with two separate adsorber beds, a first bed with silica gel and a subsequent bed with the zeolite. Following the typical procedure described above, an initial amount of 160 g hexafluoro-1,3-butadiene was purified at a flow rate of 5.0 g/l a pressure measured at pressure gauge **P2** of 100 mbar (abs.) and a temperature of 23 °C measured at thermocouple **T2**.

The results of the analyses of the crude hexafluoro-1,3-butadiene from tank **C1** and the purified hexafluoro-1,3-butadiene from tank **C2** are shown in table 1.

**Table 1: Analysis results**

| ***Compounds*** | ***Tank C1*** | ***Tank C2*** |
|---|---|---|
| *total hydrohalogenocarbon impurities (ppm)* | 588 | 117 |
| *isopropanol (ppm)* | 9 | *not detected* |

## Claims

1. A process for the purification of hexafluoro-1,3-butadiene comprising a step wherein a gaseous mixture comprising hexafluoro-1,3-butadiene is contacted with at least two different adsorbents to purify said gas mixture, wherein the at least two different adsorbents each have an average pore size of above 6 Å.

2. The process according to claim 1 wherein the at least two different adsorbents each have an average pore size of above 6 Å and equal to or lower than 100 Å.

3. The process according to claim 1 or 2 wherein at least one of the at least two different adsorbents is silica gel, preferably a silica gel with an average pore size of 10 to 50 Å.

4. The process according to any one of the aforementioned claims wherein at least one of the at least two different adsorbents is a zeolite, preferably a molecular sieve 13X zeolite.

5. The process according to any one of the aforementioned claims wherein the at least two different adsorbents comprise one zeolite and one silica gel.

6. The process according to claim 5 wherein the gaseous mixture is first purified with the silica gel and subsequently purified with the zeolite.

7. The process according to any one of the aforementioned claims wherein the gaseous mixture is contacted with the at least two different adsorbents at an initial pressure of equal to or above 100 mbar (abs.) and equal to or below 2000 mbar (abs.).

8. The process of any one of the aforementioned claims wherein the gaseous mixture is contacted with the at least two different adsorbents at an initial temperature of equal to or above 5°C and equal to or below 40°C.

9. The process according to any one of the aforementioned claims wherein the gaseous mixture is contacted with the at least two different adsorbents at a flow rate of equal to or above 2 g/min and equal to or below 200 g/min.

10. The process according to any one of the aforementioned claims wherein the at least two different adsorbents are present in different zones in the same adsorber cartridge.

11. The proces according to any one of claim 1 to 9 wherein the at least two different adsorbents are present in two different adsorber cartridges.

12. The process according to any one of the aforementioned claims wherein at least one of the adsorbents is pre-treated before being contacted with the gaseous mixture.

13. A process for the production of a gas mixture comprising the process according to any one of the aforementioned claims and subsequently, mixing the purified hexafluoro-1,3-butadiene with a further gas selected from the group consisting of an inert gas, oxygen and another fluorinated etching gas.

14. A gas mixture comprising hexafluoro-1,3-butadiene and at least one further gas selected from the group consisting of an inert gas, oxygen and another fluorinated etching gas.

15. A process for the production of a semiconductor material, a solar panel, a flat panel or a microelectromechanical system, or a process for cleaning the chamber of an apparatus used for semiconductor manufacturing, using the hexafluoro-1,3-butadiene purified according to any of the claims 1 to 12 or the gas mixture according to claim 14.
